Europäisches Patentamt

**European Patent Office**

**Office européen des brevets**

(11) Publication number: **0 170 262**
**A2**

(12) **EUROPEAN PATENT APPLICATION**

(21) Application number: **85109586.9**

(22) Date of filing: **30.07.85**

(51) Int. Cl.⁴: **A 61 F 2/24**

(30) Priority: **31.07.84 JP 160626/84**

(43) Date of publication of application:
**05.02.86 Bulletin 86/6**

(84) Designated Contracting States:
**DE FR GB NL SE**

(71) Applicant: **TERUMO KABUSHIKI KAISHA trading as TERUMO CORPORATION**
**44-1, 2-chome, Hatagaya Shibuya-Ku**
**Tokyo 151(JP)**

(72) Inventor: **Yokoyama, Yoshikane**
**3-28-21-106, Todoroki**
**Setagaya-ku Tokyo(JP)**

(72) Inventor: **Ohkawa, Yasunori**
**11-10-101, Serigaya 1-chome**
**Kounan-ku Yokohama-shi(JP)**

(72) Inventor: **Kijima, Toshihiko**
**2517 Oomiya**
**Fujinomiya-shi Shizuoka-ken(JP)**

(72) Inventor: **Kusakabe, Masahiro**
**3-381, Mineoka-cho**
**Hodogaya-ku Yokohama-shi(JP)**

(74) Representative: **Henkel, Feiler, Hänzel & Partner**
**Möhlstrasse 37**
**D-8000 München 80(DE)**

(54) **Prosthetic valve.**

(57) A prosthetic valve comprising an annular member (4) to be fitted to the inner wall of a blood passage, a plurality of columns (6) each having one end connected to the inner wall (2) of the annular member (4) and their other ends fixed together in a point, and a flexible membrane (10) mounted between the columns (6) and having a skirt portion (10a) which can, alternately, apply itself to or leave the inner wall surface of the annular member (4) depending upon the balance of blood pressure on both sides of the membrane (10), thereby allowing for a unidirectional blood flow.

F I G. 3

- 1 -

## Prosthetic valve

The present invention relates to a prosthetic valve for mounting, as a surgical prosthesis, in a blood circuit, an artificial heart, or an aortic or pulmonary valve.

In a conventional ball valve or inclined disk valve used as a prosthetic valve as described above, a ball or movable disk member is movably arranged in an opening of a normally circular suture ring. A column frame is arranged around the movable member to prevent its accidental removal. When the valve is closed, the movable member is sealed to the inner wall of the suture ring to prevent reverse flow of blood. When the valve is opened, the movable member is shifted parallel to the axis or inclined thereto to thereby form a blood path.

In a conventional ball or inclined disk valve of this type, an unnatural reverse flow of blood may be caused, or a loud sound produced due to the hard material normally used.

A prosthetic valve having a trilobed valve structure with properties similar to natural valves, due to the use of a biomembrane, is also known. An Ionescu-Shieley valve, a Carpenter-Edwards valve (Japanese Patent Disclosure (Kokai) No. 50-806965) are examples of such a valve. However, these type of valves have complex structures and are difficult to manufacture, resulting in high cost.

The present invention has been made in consideration of this, and has as its object to provide a prosthetic valve which can easily and reliably close so that reverse flow of blood does not occur, which produces only a small valve sound and which is easy and less costly to manufacture.

In order to achieve the above object of the present invention, there is provided a prosthetic valve comprising an annular member, a plurality of columns each having an end connected, at equal intervals, to an inner wall surface of the annular member while the other ends are fixed together in a point to the side of the annular member, and a flexible, movable valve membrane mounted between the columns and having a skirt portion applied to the inner wall surface of the annular member, wherein, when an external pressure acting on the movable valve membrane is higher than that acting internally, the skirt portion of the valve membrane is flexed inward so as to define a flow path between the valve membrane and the inner wall surface of the annular member, whereas, when the internal pressure overcomes the external pressure, the skirt portion of the valve membrane is reapplied to the inner wall surface of the annular member so as to close the flow path.

This invention can be more fully understood from the following detailed description when taken in conjunction with the accompanying drawings, in which:

Fig. 1 is a perspective view of a prosthetic valve according to an embodiment of the present invention;

Fig. 2 is a plan view of the prosthetic valve shown in Fig. 1 and viewed from below;

Fig. 3 is a longitudinal sectional view of the prosthetic valve shown in Fig. 1;

Fig. 4A is a plan view showing the prosthetic valve of Fig. 1 in an open state;

Fig. 4B is a sectional view corresponding to

Fig. 4A;

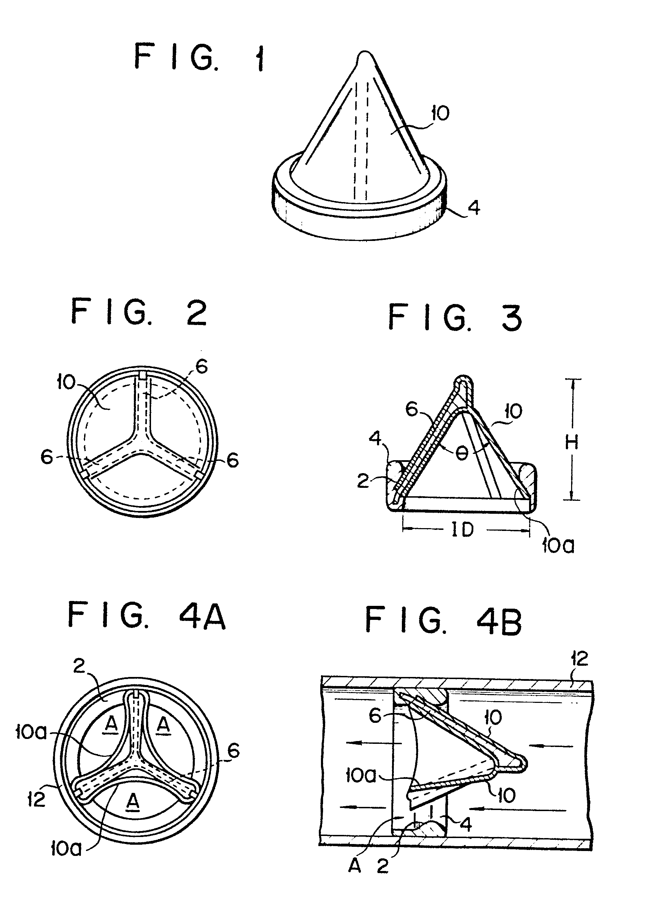

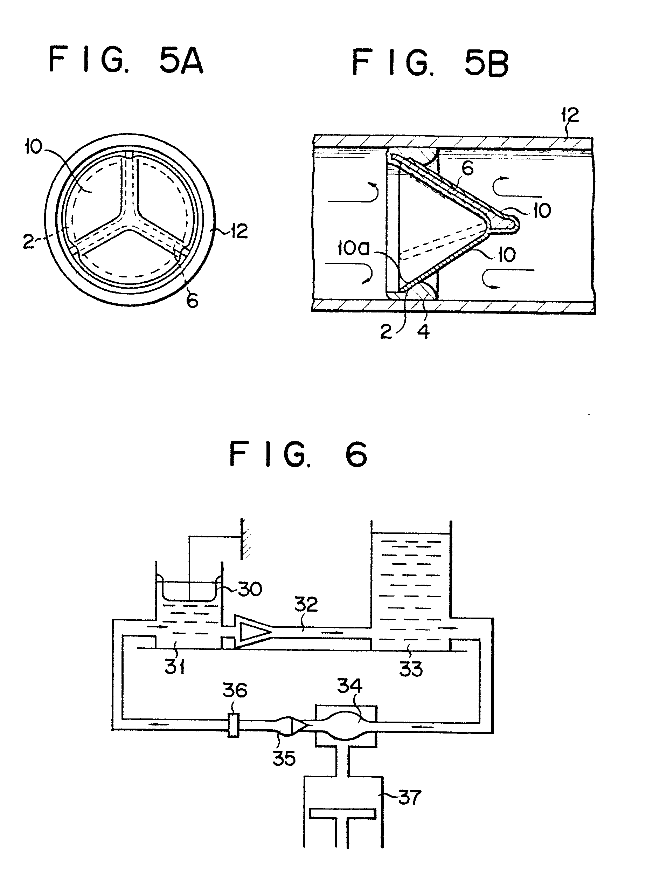

Fig. 5A is a plan view showing the prosthetic valve of Fig. 1 in a closed state;

Fig. 5B is a sectional view corresponding to Fig. 5A;

Fig. 6 is an experiment circuit diagram for testing the operation of the prosthetic valve;

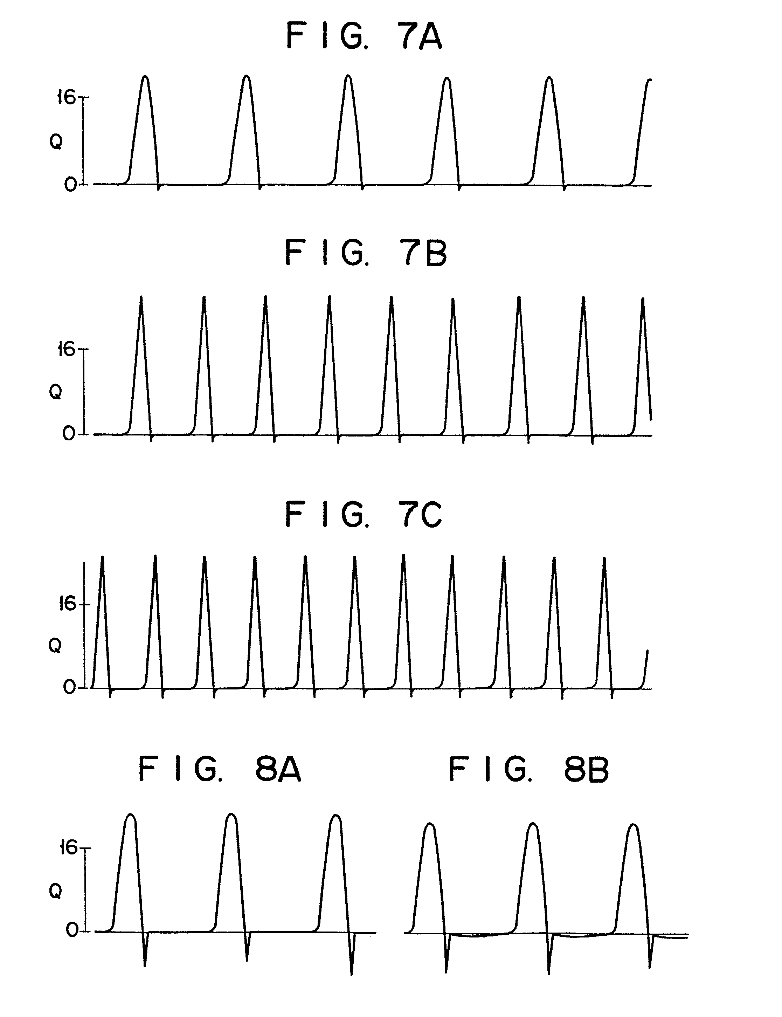

Figs. 7A to 7C, and Figs. 8A and 8B are graphs showing the operation characteristics of the prosthetic valve;

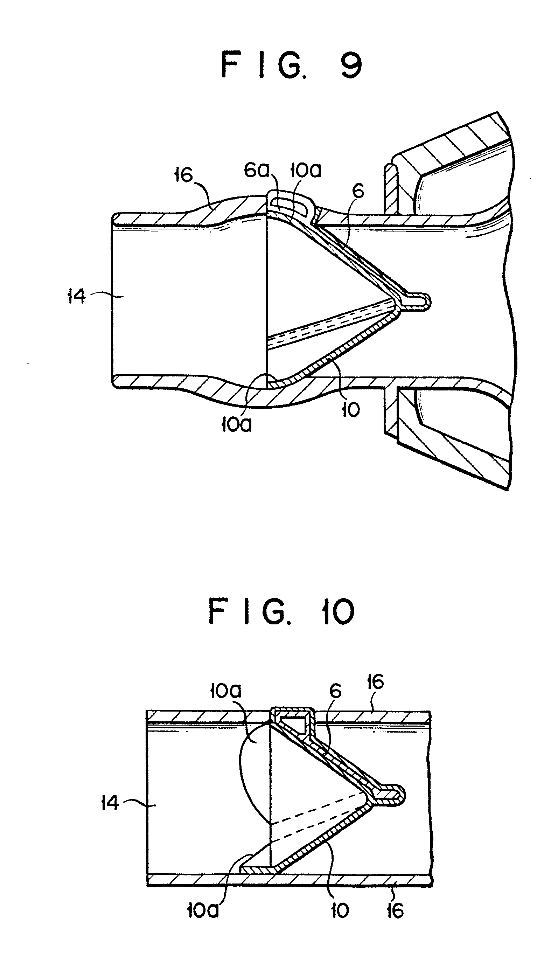

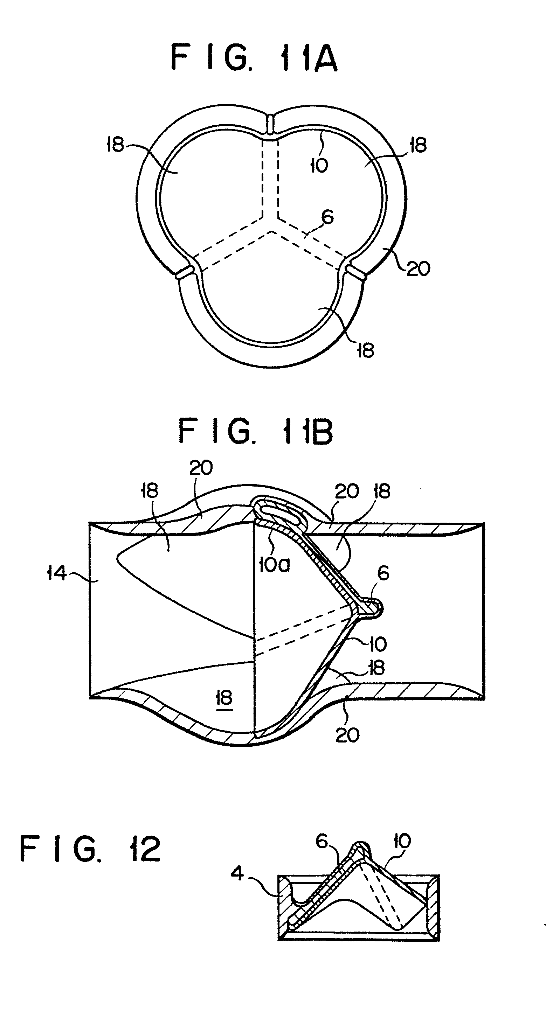

Figs. 9 and 10 are sectional views showing modifications according to the present invention;

Fig. 11A is a plan view showing another modification of the present invention;

Fig. 11B is a sectional view corresponding to Fig. 11A; and

Fig. 12 is a sectional view showing still another modification according to the present invention.

A preferred embodiment of the present invention will be described with reference to the accompanying drawings.

Figs. 1 to 3 show a prosthetic valve according to an embodiment of the present invention. Fig. 1 is a perspective view of the valve, Fig. 2 is a bottom view thereof, and Fig. 3 is a sectional view along a central portion thereof. As can be seen from Fig. 3, the prosthetic valve consists of a suture ring 4, three columns 6 and a flexible, movable valve membrane 10. The suture ring 4 has a tapered inner wall surface 2. As can be seen from Figs. 1 to 3, the columns 6 each have one end fixed to the inner wall surface 2 at equal intervals and their other ends connected in a point to thereby form a pyramid or a cone at one side of the ring 4. The membrane 10 is supported between the columns 6 and has its skirt portion 10a applied to the inner wall surface 2 of the suture ring 4.

The suture ring 4 serves as a mount medium and a valve seat when the prosthetic valve is used in an extracorporeal circuit, or mounted in a patient's body. This suture ring 4 can consist of a synthetic resin having a certain degree of elasticity, e.g., polyurethane, silicone rubber, polycarbonate, polyvinyl chloride, teflon or the like.

The columns 6 support and divide the membrane 10 so as to facilitate movement of the membrane 10. Normally, three columns are used to form a triangular pyramid. When the prosthetic valve is intended for embedding in a patient's body, the pyramid or cone should be, preferably, of a low height so as not to damage the patient's tissue. Further, the height of the pyramid or cone should not, preferably, exceed the inner diameter of the suture ring 4, being, in practice, 60 to 80% of the inner diameter (normally 15 to 30 mm) of the suture ring 4. Therefore, the vertex angle should, preferably, exceed 60 degrees. When the vertex angle is too large, the open area becomes too small to provide a high blood resistance. For this reason, the vertex angle should, preferably, be below 90 degrees. When the prosthetic valve is used for extracorporeal applications, the vertex angle of the triangular pyramid is not so particularly limited. However, the vertex angle should, preferably, be 45 degrees or more, as even if the vertex angle is decreased below 45 degrees, though the open area (the area in a fully open state) is itself not increased beyond a certain limit, the height of the valve is increased, rendering the valve too large. The material for the columns 6 can be a metal having a sufficient rigidity, e.g., stainless steel or titanium, or a synthetic resin such as polypropylene or polyacetal.

The membrane 10 is pivoted or distorted between the columns 6 in accordance with the direction of blood pressure, and provides a valve action in conjunction with the suture ring 4. The membrane 10 consists of a material having a sufficient mechanical strength, a suitable elasticity and an anti-thrombotic property. Examples of such a material include an elastomer, e.g., polyurethane, silicone rubber, polyolefin rubber or natural rubber, or a biomembrane such as a cardiac vesicle membrane, or the like, from a cow, or a dura mater membrane, or the like, of a human kind.

When a material other than a biomembrane is used, it is coated with an anti-thrombotic material, e.g., silicone, polyhydroxyethylacrylate, polyhydroxy-methylacrylate, or a block copolymer of hydroxyethylacrylate and styrene.

A method of manufacturing a prosthetic valve as described above will be described below. First, a embrane material solution is applied on the surface of a mold, having substantially a triangular pyramid shape, so as to form a coating layer. Columns are then set at predetermined positions on the coating layer. After applying the membrane material solution again, the periphery of the coating layer is trimmed and the coating layer is removed from the mold. The membrane is assembled with a separately molded suture ring, and the columns are adhered with an adhesive. (It should be noted that the columns and the membrane can be integrally molded from a single material.)

The operation of the prosthetic valve prepared in this manner will be described. As shown in Fig. 5B, the prosthetic valve is fixed in a suitable position in a blood path 12 such that the blood flow is through the suture ring 4, and so that the pointed end of the membrane is directed downstream. When the heart contracts (expands) and the membrane upstream pressure exceeds the downstream pressure, the membrane 10 collapses due to the forward pressure difference as shown in Figs. 4A and 4B. Then, a gap A is defined between the suture ring 4 and the skirt portion 10a of the membrane 10, so that blood flows to the downstream side.

When the heart expands (contracts) and the membrane downstream pressure exceeds the upstream pressure, the membrane 10 is subject to the pressure acting toward the upstream side such that the skirt portion 10a is applied to the tapered surface 2 of the suture ring 4, thereby preventing reverse flow of blood. When the membrane consists of a material having a high elasticity, the elasticity overcomes the force corresponding to the pressure difference before the downstream pressure exceeds the upstream pressure, thereby starting the return operation (close operation) of the membrane 10.

A comparison will be made as to reliability, leakage, and reverse flow in repeated open/close tests of a prosthetic valve of the present invention and conventional commercially available prosthetic valves.

The prosthetic valve according to the present invention tested had a shape as shown in Fig. 3. More specifically, the suture ring 4 consisted of an acrylic resin and had an inner diameter ID of 23 mm, while the columns 6 were of stainless steel. The membrane had a thickness of about 250 μm and consisted of a polyurethane elastomer (available from Nippon Polyurethane K.K.). The valve had a height H of 23 mm and a vertex angle θ of 60 degrees.

The conventional prosthetic valves tested were an SJM valve (St. Jude Medical valve) having an inner diameter of 23 mm, and a Björk-Shiley valve having an inner diameter of 24 mm (e.g., see "Ko to Jun", Vol. 27, No. 8, August 1979, pp. 825 - 837).

The test circuit used was a pulse duplicator which kinetically simulated the blood vessel system of a patient as shown in Fig. 6. Referring to Fig. 6, reference numeral 30 denotes a peripheral resistance element; 31, an arterial volume element; 32, an inertia element; 33, a vein volume element; 34, an artificial heart; 35, a prosthetic valve; 36, an electromagnetic flowmeter and 37, an artificial heart driver unit.

The obtained results are shown in Figs. 7A, 7B, 7C, 8A and 8B. In these figures, reference numeral Q denotes an aortic blood flow rate. In the case of the prosthetic valve according to the present invention, as shown in Figs. 7A to 7C, the parameters of the device are set to correspond to hard blood vessels and high resistance. The number of heart beats in this case is 60 to 120 (beats/min): 60 beats/min for Fig. 7A, 90 beats/min for Fig. 7B and 120 beats/min for Fig. 7C. Fig. 8A shows the results obtained with the SJM valve, and Fig. 8B shows the results obtained with the Björk-Shiley valve.

It can be seen from Figs. 7A to 7C that when the prosthetic valve according to the present invention is used, the valve operates regularly, and reverse flow or blood leakage upon valve closing operation (curve portion below the line $Q = 0$) occurs less often than in the case of the conventional valves (Figs. 8A and 8B). Furthermore, the valve sound was equivalent to a natural valve.

The present invention is not limited to the embodiment shown in Figs. 1 to 3, and various changes and modifications may be made within the spirit and scope of the appended claims. For example, as shown in Fig. 9, a prosthetic valve can be connected integrally in a flow path 14 of an artificial heart through annular proximal ends 6a of the columns 6, while, similarly, a skirt portion 10a of the membrane 10 and the inner proximal ends of columns 6 may be bent inward so as to provide better attachability to the inner wall surface of the flow path 14. In this case, a tube portion 16 of the artificial heart has an equivalent function to that of the suture ring in the above embodiment.

In a modification shown in Fig. 10, a prosthetic valve is formed integrally with a flow path 14 of an artificial heart in almost the same manner as that shown in Fig. 9. However, this modification is different from that shown in Fig. 9 in that a skirt portion 10a of the membrane 10 projects in an arcuated shape between each two adjacent columns 6 so as to provide a better seal with the inner wall surface of the flow path.

In a modification shown in Figs. 11A and 11B, a recess 18 like a bursa cavity is formed between each of two adjacent columns 6 to form a flow path 14. A turbulent flow is formed upon the closing operation of the valve so as to provide a cleaning effect of the membrane 10. In this case, a pipe 20 included with the recesses 18 is equivalent to the suture ring of the embodiment described above.

In a modification shown in Fig. 12, in order to decrease the valve height, three membrane portions 10, defined by columns 6, are not formed into a single cone-shape, but three cone-like surfaces having different angles with respect to the central axis of a suture ring 4 are combined as shown in Fig. 11A. This prosthetic valve has an open area larger than that of a prosthetic valve having the shape of a single cone-shape. Unlike in Figs. 11A and 11B, in the valve shown in Fig. 12, the outer portions of the membrane are cut by a cylindrical plane, and the skirt portions of the membrane are shorter than in the modification shown in Figs. 11A and 11B. In order to improve its application to the cylinder, the skirt portions may be extended as shown in Fig. 10.

In summary, a prosthetic valve according to the present invention can close easily and reliably, causes only infrequent and inconsequential reverse flow and leakage, has a small valve sound, and is easy and inexpensive to manufacture.

0170262

Claims:

1. A prosthetic valve comprising an annular member (4), a plurality of columns (6) each having one end connected to an inner wall (2) surface of said annular member (4) at equal intervals and their other ends fixed together in a point, and a flexible, movable valve membrane (10) mounted between said columns (6) and having a skirt portion applied to the inner wall (2) surface of said annular member (4), wherein, when an external pressure acting on said movable valve membrane (10) is higher than that acting internally, said skirt portion of said valve membrane (10) is flexed inward so as to define a flow path between said valve membrane (10) and the inner wall surface of said annular member (4), whereas, when the internal pressure overcomes the external pressure, said skirt portion of said valve membrane (10) is reapplied to the inner wall surface of said annular member (4) so as to seal the flow path.

2. A prosthetic valve according to claim 1, characterized in that a portion of said membrane (10) between each two adjacent columns (6) projects outward in an arcuated shape.

3. A prosthetic valve according to claim 1, characterized in that said columns (6) are three in number (6).

4. A prosthetic valve according to claim 1, characterized in that said annular member (4) is bulged between the proximal ends of the columns (6).

5. A prosthetic valve according to claim 1, characterized in that said columns (6) and said valve membrane (10) are integrally formed of an identical raw material.

6. A prosthetic valve according to claim 1, characterized in that each proximal end of the column (6) is buried in the annular member (4).

7. A prosthetic valve according to claim 1, characterized in that the annular member (4) constitutes
a portion of a blood passage in an artificial heart.

F I G. 1

F I G. 2

F I G. 3

F I G. 4A

F I G. 4B

FIG. 5A

FIG. 5B

FIG. 6

# F I G. 7A

# F I G. 7B

# F I G. 7C

# F I G. 8A          F I G. 8B

0170262

# F I G.  9

# F I G.  10

0170262

# F I G. 11A

# F I G. 11B

# F I G. 12